# FASCICULE DE BREVET EUROPEEN

(11) **EP 0 723 974 B1**
(45) Date de publication et mention de la délivrance du brevet: **16.08.2001**
(21) Numéro de dépôt: 96400136.6
(22) Date de dépôt: 22.01.1996
(51) Int. Cl.: C07K 5/06

(54) **Dérivés de mercaptoalcanoyldipeptides, leur procédé de préparation et les compositions pharmaceutiques qui les contiennent**
Merkaptoalkanoyldipeptide Derivate, Verfahren zu deren Herstellung und diese enthaltende pharmazeutische Zusmmensetzungen
Derivatives of mercaptoalkanoyldipeptides, their preparation and pharmaceutical compositions containing them

(30) Priorité: 23.01.1995 FR 9500694
(43) Date de publication de la demande: 31.07.1996
(73) Titulaire: ADIR ET COMPAGNIE, 92415 Courbevoie Cédex (FR); INSTITUT NATIONAL DE LA SANTE ET DE LA RECHERCHE MEDICALE (INSERM), 75654 Paris Cédex 13 (FR)
(72) Inventeur: Roques, Bernard P., F-94410 Saint-Maurice (FR); Fournie-Zaluski, Marie-Claude, F-75020 Paris (FR)

(56) Documents cités:
- EP-A- 0 059 966
- EP-A- 0 524 553
- WO-A-94/17036
- GB-A- 2 159 160

## Description

La présente invention concerne de nouveaux dérivés de mercaptoalcanoyldipeptides inhibiteurs mixtes de l'endopeptidase neutre (NEP) et de l'enzyme de conversion de l'angiotensine (ACE), leur procédé de préparation et les compositions pharmaceutiques qui les contiennent.

Les nouveaux dipeptides selon l'invention présentent la remarquable propriété d'inhiber à la fois l'endopeptidase neutre (E.C. 3.4-24.11) qui provoque l'inactivation du peptide natriurétique auriculaire et la peptidyldipeptidase A (E.C. 3.4-15.1) qui génère l'angiotensine II. Ils se comportent donc comme des inhibiteurs mixtes de l'endopeptidase neutre (NEP) et de la peptidyldipeptidase A (ACE) capables de potentialiser les effets natriurétiques, diurétiques et vasodilatateurs du peptide natriurétique auriculaire et de bloquer les effets hypertenseurs provoqués par l'angiotensine II.

De nombreux produits possédant l'une ou l'autre de ces activités sont connus.

Dans le brevet EP-B-38758 sont décrits des produits qui sont des inhibiteurs de l'endopeptidase neutre dénommée "enképhalinase", car l'enzyme dégrade les enképhalines qui sont des ligands endogènes des récepteurs morphiniques. Ces inhibiteurs sont, par conséquent, utiles comme analgésiques.

Ainsi, dans *Nature*, ***288,*** (1980), 286-288, B.P. Roques *et al*. ont montré que la (*R*,*S*)-(2-mercaptométhyl-3-phénylpropionyl)glycine (thiorphan) a un pouvoir inhibiteur à une concentration nanomolaire et se comporte comme un analgésique en potentialisant l'action des enképhalines, peptides opiacés endogènes.

D'autres inhibiteurs de l'enképhalinase, doués de propriétés analgésiques, font l'objet, par exemple, des brevets FR-B-2 556 721 et EP-B-136 883.

Dans les brevets US-A-4,053,651, US-A-4,684,660 et EP 059966 sont décrits des inhibiteurs de la peptidyldipeptidase A, utiles comme anti-hypertenseurs.

Koehn *et al*., (*J*. *Biol. Chem*., ***262,*** (1987), 11623-11627) et S.L. Stephenson and A.J. Kenny, (*Biochem. J*., ***243,*** (1987), 183-187) ont rapporté que le peptide natriurétique auriculaire libéré par le coeur, particulièrement en cas d'insuffisance cardiaque, et qui augmente la natriurèse et diurèse et manifeste ainsi un effet vasodilatateur, est inactivé par l'enzyme E.C. 24.11 périphérique. Ainsi, l'inhibiteur d'endopeptidase neutre, le thiorphan et certains de ses dérivés, sont capables d'augmenter la demi-vie du peptide natriurétique auriculaire circulant et d'abaisser la pression sanguine chez le rat (G. Olins *et al*., *Mol. Cell. Endocrinol*., ***61,*** (1989), 201-208 ; A.A. Seymour *et al*., *Hypertension, **14,*** (1989), 87-97).

Cependant, aucun des inhibiteurs connus de l'endopeptidase neutre ne possède une activité anti-hypertensive intrinsèque et un inhibiteur de l'endopeptidase neutre tel que le thiorphan, dans des essais cliniques, produit une natriurèse et une diurèse sans aucun effet hypotenseur significatif et sans diminution de surcharge cardiaque.

Toutefois, dans le brevet US-A-4,879,309 sont décrits des composés de formule : 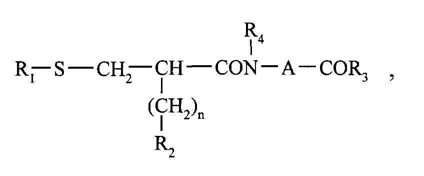 qui sont utiles pour augmenter la natriurèse et la diurèse et pour réduire la pression sanguine.

Des inhibiteurs mixtes de NEP et d'ACE sont décrits dans les brevets FR-B-2 682 952, FR-A-2 679 564 et WO 94/17036. Les composés décrits admettent pour formules
- pour le brevet FR-B-2 682 952 : 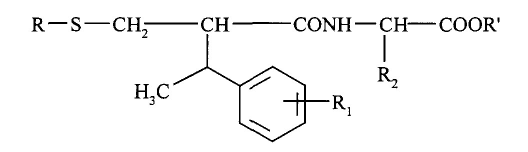
- pour les brevets FR-A-2 679 564 et WO 94/17036:

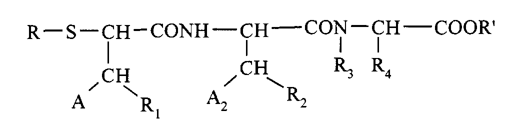

La présente invention concerne de nouveaux dipeptides qui inhibent à la fois l'endopeptidase neutre et la peptidyldipeptidase A à des concentrations très faibles. Les composés selon l'invention diffèrent des composés de l'art antérieur, en particulier des brevets US-A-4,879,309 et FR-A-2 679 564, par le fait que tous les composés de la présente invention possèdent en partie C-terminale un imino-acide à 5 ou 6 chaînons substitué respectivement en position 5 ou 6 par un radical phényle ou phényle substitué. De plus, cet imino-acide, très encombrant, impose l'enchaînement Gly-imino-acide pour qu'une excellente activité inhibitrice soit obtenue sur les deux enzymes. Ce type d'enchaînement conduit à des composés qui possèdent des pouvoirs inhibiteurs très puissants sur les deux enzymes (K_{I} de l'ordre de 10⁻⁹ M), des durées d'action très longues et des biodisponibilités adéquates pour bloquer à la fois l'enzyme de conversion qui se trouve majoritairement dans les vaisseaux et l'endopeptidase neutre qui dégrade le peptide natriurétique, principalement au niveau du tubule proximal rénal.

Les nouveaux composés selon l'invention possèdent la double propriété d'inhiber à la fois l'endopeptidase neutre et la peptidyldipeptidase A. De ce fait, l'effet hypotenseur obtenu avec ces inhibiteurs mixtes est plus important que celui obtenu avec les inhibiteurs de l'endopeptidase neutre et de la peptidyldipeptidase A, utilisés seuls ou en mélange. De plus, les composés de l'invention présentent une inhibition de l'endopeptidase neutre et de la peptidyldipeptidase A équilibrée. En effet, les valeurs d'IC₅₀ obtenues pour un composé donné sont sensiblement identiques pour l'endopeptidase neutre et la peptidyldipeptidase A. Ceci montre la supériorité des composés de l'invention par rapport à ceux de l'art antérieur décrits dans les brevet US-A-4,879,309 et FR-A-2 679 564.

Ainsi, les composés de la présente invention sont très efficaces dans le traitement des défaillances cardiaques congestives, et de divers types d'hypertensions, dans le traitement de l'athérosclérose, de l'ischémie cardiaque, des défaillances rénales chroniques, des accidents cardiaques liés à une affection pulmonaire. Ils sont également efficaces pour réduire les effets secondaires dus au traitement par la cyclosporine.

Plus particulièrement, la présente invention concerne des composés de formule générale (I) : 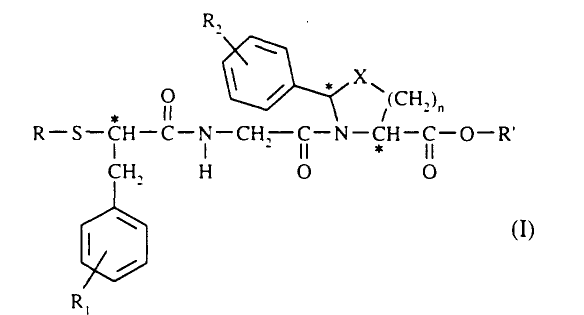 dans laquelle :
- **R** est choisi parmi l'hydrogène, un radical acyle contenant de 1 à 6 atomes de carbone en chaîne droite ou ramifiée, le radical benzoyle, naphtoyle, adamantoyle et le radical 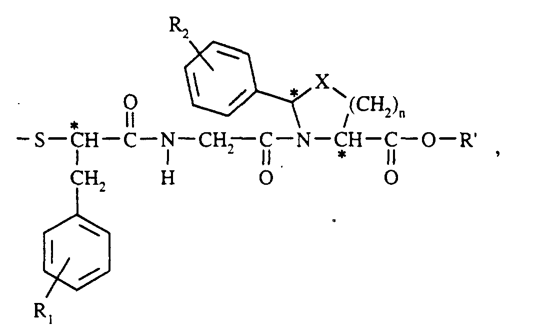 dans lequel R₁, R₂, R', X et n sont définis ci-après,
- **R**_{**1**} et **R**_{**2**} identiques ou différents, sont choisis, indépendamment l'un de l'autre, parmi l'hydrogène, le radical hydroxy, un radical alkyle contenant de 1 à 6 atomes de carbone en chaîne droite ou ramifiée, un radical alkoxy contenant de 1 à 6 atomes de carbone, en chaîne droite ou ramifiée, un atome d'halogène, choisi parmi fluor, chlore, brome et iode, un groupement nitro, un groupement amino, le radical carboxy, un radical alkoxycarbonyle contenant de 1 à 6 atomes de carbone en chaîne droite ou ramifiée, le radical benzyloxycarbonyloxy, le radical phosphonate, le radical sulfonate, le radical SO₂-NH₂ et le radical SO₂-NH-alkyle, dans lequel le terme alkyle désigne un groupement hydrocarboné saturé contenant de 1 à 6 atomes de carbone, en chaîne droite ou ramifiée,
- **R'** est choisi parmi l'hydrogène, un radical alkyle contenant de 1 à 20 atomes de carbone en chaîne droite ou ramifiée, un radical aralkyle et un radical cycloalkylalkyle,
- **X** est choisi parmi l'oxygène, le soufre, le groupement CH₂ et le groupement NH,
- **n** est choisi parmi les valeurs entières 1 et 2,
étant entendu que :
- "radical aralkyle" désigne un radical formé d'un groupement aryle, choisi parmi phényle et naphtyle, lié à un radical alkyle contenant de 1 à 6 atomes de carbone en chaîne droite ou ramifiée,
- "radical cycloalkylalkyle" désigne un radical formé d'un groupement cycloalkyle contenant de 3 à 8 atomes de carbone, lié à un radical alkyle contenant de 1 à 6 atomes de carbone en chaîne droite ou ramifiée,
ainsi que leurs formes stéréoisomériques à l'état pur ou en mélange et leurs éventuels sels d'addition, pharmaceutiquement acceptables.

La présente invention s'étend également au procédé de préparation des composés de formule (I), caractérisé en ce que le dipeptide de formule (II) : 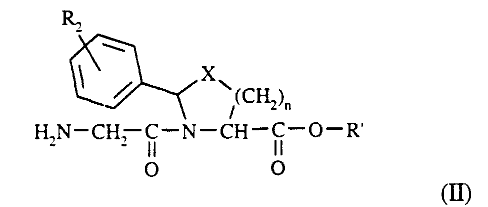 dans laquelle R₂, R', X et n sont tels que définis pour le composé de formule (I),
est soumis à une réaction d'acylation au moyen d'un acide de formule (III) : 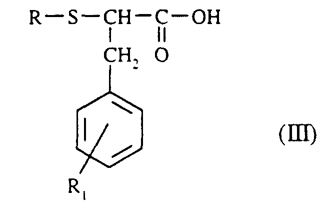 dans laquelle R et R₁ sont tels que définis pour le composé de formule (I),
dans les conditions habituelles utilisées en synthèse peptidique et décrites, par exemple, par Bodansky *et al*., ("*Peptide Synthesis*", J. Wiley and Sons Edit).

Les dipeptides de formule (II) sont obtenus par condensation du chlorure d'acyle de la Z-glycine de formule (IV) : 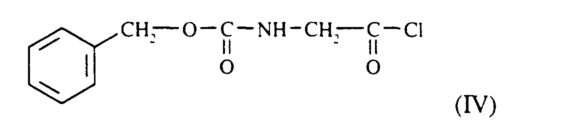 avec un ester de formule (V) : 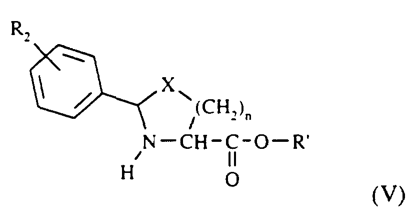 dans laquelle R₂, R', X et n sont tels que définis précédemment, suivie d'une hydrogénation catalytique.

Le composé de formule (III) peut être préparé à partir de l'α-amino acide correspondant par déamination halogénante selon Fisher *et al*., (*Ann*., ***357,*** (1907), 1-24), suivie d'une substitution nucléophile de l'atome d'halogène.

Les composés de formule (I) peuvent également être obtenus par condensation, par exemple à l'aide du réactif de Castro, des composés de formule (V) avec les pseudodipeptides de formule (VI) : 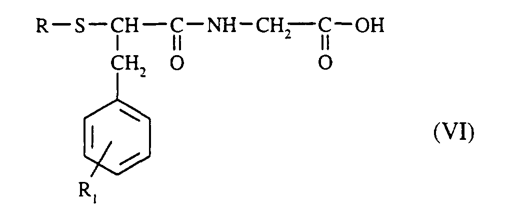 dans laquelle R et R₁ sont tels que définis précédemment.

Les pseudodipeptides de formule (VI) sont obtenus par couplage, selon des méthodes classiques utilisées en synthèse peptidique, des acides de formule (III) avec l'ester *tertio*-butylique de la glycine, puis déprotection de la fonction acide par l'acide trifluoroacétique.

Les composés de formule (V) sont obtenus selon les méthodes décrites, par exemple, par J.N. Barton et *al*., (*J. Med*. *Chem*., ***30***, (1990), 1606-1615).

Les composés de formule (I) dans laquelle R' représente l'hydrogène peuvent former des sels pharmaceutiquement acceptables avec des bases minérales ou organiques. Parmi les bases minérales ou organiques utilisées pour former ces sels, on citera plus particulièrement, et de manière non limitative, les hydroxydes d'ammonium, de sodium ou de calcium, la N-méthyl-D-glycamine, la lysine, l'arginine ou la dicyclohexylamine.

Les composés de la présente invention peuvent, le cas échéant, exister sous forme de stéréoisomères purs ou sous forme de mélanges de stéréoisomères. Les stéréoisomères purs sont aisément accessibles par l'homme du métier, à partir des mélanges de stéréoisomères selon des techniques classiques de séparation, ou par synthèses asymétriques des produits de départ.

Les atomes de carbone marqués d'un astérisque (∗) dans la formule (I) sont des centres d'asymétrie. Les composés préférés de la présente invention sont ceux pour lesquels :
- l'atome de carbone portant le radical 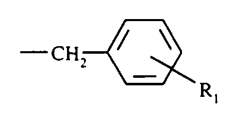 adopte la configuration S, et
- l'atome de carbone portant le radical 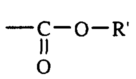 adopte la configuration S, et
- les radicaux 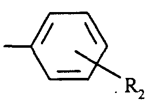 d'une part et 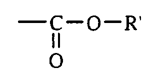 d'autre part sont disposés par rapport au plan moyen du cycle en configuration *cis.*

Les demanderesses ont découvert que les composés de l'invention sont doués de propriétés pharmacologiques très intéressantes.

Les composés de la présente invention, lorsqu'ils sont administrés à des mammifères sont utiles dans le traitement et la prévention des défaillances cardiaques congestives, des hypertensions essentielles, des hypertensions liées à une augmentation du volume sanguin, de l'insuffisance cardiaque et/ou rénale, de l'angine de poitrine et possèdent un effet protecteur sur l'épaississement de la paroi vasculaire. Ceci est dû à leur double action inhibitrice de l'endopeptidase neutre et de la peptidyldipeptidase A. De part leurs propriétés pharmacologiques, les composés de la présente invention sont également utiles dans le traitement et la prévention de l'athérosclérose, et de l'ischémie cardiaque. Ils sont également utiles dans la prévention de l'hypertrophie, de la fibrose ventriculaire gauche et du coeur pulmonaire. Des patients ayant subit des transplantations cardiaques et/ou rénales sont souvent traités à long terme par la cyclosporine. Celle-ci a pour effets secondaires une augmentation de la pression sanguine et une rétention de sel. Les composés de l'invention possèdent des propriétés s'opposant à ces effets et sont donc utiles pour réduire les effets secondaires dus à la cyclosporine.

Les inhibiteurs de la peptidyldipeptidase A sont des produits connus pour traiter certaines hypertensions : ils sont capables de bloquer l'augmentation de la pression sanguine provoquée par une augmentation de la résistance vasculaire et du volume sanguin due à la formation de l'angiotensine II à partir de l'angiotensine I.

Un autre peptide important impliqué dans la régulation de la pression artérielle est le peptide natriurétique auriculaire libéré par le coeur, qui est doté d'une propriété vasodilatatrice, et qui est capable de contrôler la diurèse et la natriurèse. Le peptide natriurétique auriculaire est dégradé par l'endopeptidase neutre dans les tissus périphériques. Les inhibiteurs d'endopeptidase neutre, tels que le thiorphan, induisent une diurèse et une natriurèse significatives chez l'homme sans faire apparaître une augmentation des taux de rénine et d'aldostérone habituellement constatée avec les diurétiques généralement utilisés en association avec les inhibiteurs d'ACE. Cependant, les effets sur la pression sanguine des inhibiteurs de l'endopeptidase neutre, utilisés seuls, sont faibles.

Les inhibiteurs mixtes de l'endopeptidase neutre et de la peptidyldipeptidase A peuvent soulager les hypertensions humaines, de diverses origines et peuvent être utilisés sans co-administration de diurétiques, et sans apport de potassium.

Les nouveaux produits de la présente invention sont très efficaces de manière préventive et curative dans le traitement des défaillances cardiaques congestives et de divers types d'hypertensions, en particulier, les hypertensions liées à une augmentation du volume sanguin, des hypertensions essentielles, de l'athérosclérose, de l'ischémie cardiaque, de l'insuffisance cardiaque et/ou rénale, du coeur pulmonaire et de l'angine de poitrine, et possèdent un effet protecteur sur l'épaississement de la paroi vasculaire en protégeant de leur inactivation la bradykinine et le peptide natriurétique auriculaire, ainsi qu'une capacité à diminuer les effets secondaires dus aux traitements par la cyclosporine.

Du fait de la double propriété des composés de l'invention d'inhiber à la fois l'endopeptidase neutre et la peptidyldipeptidase A, l'effet hypotenseur obtenu avec ces inhibiteurs mixtes est plus important que celui obtenu avec les inhibiteurs de l'endopeptidase neutre et la peptidyldipeptidase A, utilisés seuls ou en mélange.

La présente invention a également pour objet les compositions pharmaceutiques contenant les composés de formule (I), ou leurs stéréoisomères purs ou en mélange, sous forme de base ou de sels d'addition, pharmaceutiquement acceptables, en combinaison avec un ou plusieurs excipients ou véhicules inertes et non toxiques.

Parmi les compositions pharmaceutiques selon l'invention, il sera cité plus particulièrement celles qui conviennent pour l'administration orale, parentérale, nasale, rectale, perlinguale, oculaire ou respiratoire, et notamment les comprimés simples ou dragéifiés, les comprimés sublinguaux, les sachets, paquets, gélules, glossettes, les tablettes, les suppositoires, les crèmes, les pommades, les gels dermiques, les préparations injectables ou buvables, les aérosols, les gouttes oculaires ou nasales.

Le choix du support et le taux de substance active dans le support sont généralement déterminés en fonction de la solubilité et des propriétés chimiques du produit, du mode particulier d'administration et des dispositions de la pratique pharmaceutique. Par exemple, des excipients tels que le lactose, le citrate de sodium, le carbonate de calcium, le phosphate dicalcique et des agents de désintégration tels que l'amidon, les acides alginiques et certains silicates complexes associés à des agents lubrifiants tels que le stéarate de magnésium, le laurylsulfate de sodium et le talc peuvent être utilisés pour préparer des comprimés. Pour préparer une capsule, il est avantageux d'utiliser le lactose et des polyéthylèneglycols de poids moléculaire élevé. Lorsque des suspensions aqueuses sont utilisées, elles peuvent contenir des agents émulsifiants ou des agents facilitant la mise en suspension. Des diluants tels que l'éthanol, le propylèneglycol, le glycérol et le chloroforme ou leurs mélanges peuvent également être utilisés.

Pour l'administration parentérale, on utilise des suspensions ou des solutions des produits selon l'invention dans l'huile de sésame, l'huile d'arachide ou l'huile d'olive ou des solutions aqueuses de propylèneglycol ainsi que des solutions aqueuses stériles des sels pharmaceutiquement acceptables. Les solutions des sels des produits selon l'invention sont particulièrement utiles pour l'administration par injection intramusculaire ou sous-cutanée. Les solutions aqueuses, comprenant également les solutions des sels dans l'eau distillée pure, sont utilisables pour l'administration intra-veineuse sous réserve que leur pH soit convenablement ajusté et qu'elles soient judicieusement tamponnées et rendues isotoniques par une quantité suffisante de glucose ou de chlorure de sodium et qu'elles soient stérilisées par chauffage ou micro-filtration.

Les doses utilisées dans les méthodes selon l'invention sont celles qui conduisent à un maximum d'effet thérapeutique jusqu'à ce qu'une amélioration soit obtenue. En général, les doses utilisées sont celles qui sont thérapeutiquement efficaces pour abaisser la pression sanguine lors du traitement de l'hypertension. En général, les doses administrées par voie orale sont comprises entre 0,1 et 100 mg/kg, de préférence entre 1 et 10 mg/kg, et celles administrées par voie intra-veineuse sont comprises entre 0,01 et 10 mg/kg, de préférence entre 0,1 et 5 mg/kg, étant entendu que, dans chaque cas particulier, les doses seront déterminées en fonction des facteurs propres au sujet à traiter tels que l'âge, le poids, l'état de santé générale et d'autres caractéristiques qui peuvent influer sur l'efficacité du médicament.

Les composés selon l'invention peuvent être administrés aussi fréquemment qu'il est nécessaire pour obtenir l'effet thérapeutique désiré. Certains malades peuvent répondre rapidement à une dose plus ou moins forte et peuvent se satisfaire de doses de maintien beaucoup plus faibles. Pour d'autres malades, il peut être nécessaire d'avoir des traitements étalés à raison de 1 à 4 doses par jour en fonction des besoins physiologiques de chaque malade particulier. Généralement, le produit actif peut être administré oralement 1 à 4 fois par jour. Pour d'autres malades, il sera nécessaire de ne pas prescrire plus d'une à deux doses par jour.

Les composés selon l'invention peuvent être utilisés sous forme injectable dans des cas d'urgence d'hypertension aiguë. Un tel traitement peut être suivi par une perfusion intra-veineuse du produit actif de façon à obtenir et à maintenir l'effet thérapeutique recherché.

Dans la présente application, les abréviations utilisées correspondent aux produits suivants :
- PyBrOP : hexafluorophosphate de bromoxy-tripyrrolidinophosphonium
- Cbz : carbobenzoxy
- Z : benzyloxycarbonyle
- BOP : hexafluorophosphate de benzotriazol-1-yl-oxy-tris(diméthylamino)phosphonium
- DCC : dicyclohexylcarbodiimide

Les exemples suivants illustrent l'invention sans toutefois la limiter en aucune façon. Les produits de départ sont connus ou préparés à partir de modes opératoires connus.

### PREPARATION A : Ester tertio-butylique de la N-(2-acétylthio-3-phénylpropanoyl) glycine

A 5 g d'acide 2-acétylthio-3-phénylpropanoïque en solution dans 40 ml de tétrahydrofurane sont ajoutées successivement une solution de 3,7 g de chlorhydrate de l'ester *tertio*-butylique de la glycine et de 3,8 ml de triéthylamine dans 40 ml de chloroforme, une solution de 3 g de 1-hydroxybenzotriazole dans 30 ml de tétrahydrofurane et une solution de 6,9 g de DCC dans le trichlorométhane. Après agitation une nuit à température ambiante, le mélange réactionnel est filtré et évaporé à sec. Le résidu, repris dans l'acétate éthyle, est successivement lavé par une solution à 10% d'acide citrique, de l'eau, une solution à 10% d'hydrogénocarbonate de sodium, de l'eau, une solution saturée de chlorure de sodium puis est séché sur sulfate de sodium. Après filtration et évaporation à sec, 7,5 g d'une huile sont obtenus.

Rendement : 98 % R_{f} : 0.65 (n-hexane/acétate d'éthyle, 2:1)

### PREPARATION B : N-(2-Acétylthio-3-phénylpropanoyl)glycine

7.5 g du composé obtenu dans la préparation A sont dissous dans 10 ml de dichlorométhane. 17 ml d'acide trifluoroacétique sont ajoutés à 0°C. Après 30 minutes à 0°C, le mélange est agité 3 heures à température ambiante, puis évaporé à sec. Le résidu est repris trois fois par un mélange 50:50 éther éthylique/n-hexane, pour fournir 6,2 g d'une huile correspondant au produit attendu.
Rendement : 97%
R_{f} : 0,53 (dichlorométhane/méthanol, 9:1)

### PREPARATION C : Acide 2-acétylthio-3-(para-benzyloxycarbonyloxyphényl)-propanoïque

A 10 g d'acide 2-bromo-3-(*para*-benzyloxycarbonyloxyphényl)propanoïque (préparé selon K. Koga *et al*. *Chem. Pharm. Bull.,* **26,** 178 (1978)), en solution dans l'hydroxyde de sodium 1M, est ajoutée une solution de thioacétate de potassium (préparé à partir de 2,5 ml d'acide thioacétique et 2 g de carbonate de potassium dans l'eau). Le mélange est agité pendant 2 jours à température ambiante. Après acidification par de l'acide chlorhydrique 1N, la phase aqueuse est extraite trois fois par l'acétate d'éthyle, lavée par une solution saturée de chlorure de sodium et séchée sur sulfate de sodium. Après filtration et évaporation à sec, une huile jaune est obtenue qui est purifiée par chromatographie sur gel de silice (éluant : n-hexane/acétate d'éthyle/acide acétique, 8:2:0,5). 6,4 g d'un produit huileux sont obtenus.
Rendement : 65%
R_{f} : 0,49 (n-hexane/acétate d'éthyle/acide acétique, 6:4:0,5).

### PREPARATION D : Ester tertio-butylique de la N-[2-acétylthio-3-(para-benzyloxy-carbonyloxyphényl)propanoyl]glycine

1,6 g du composé obtenu dans la préparation C sont condensés dans les conditions de la préparation A avec 0,72 g de l'ester *tertio*-butylique de la glycine. 2 g d'une huile jaune pâle sont obtenus.
Rendement : 96%
R_{f} : 0.57 (hexane/acétate d'éthyle, 1:1)

### PREPARATION E : N-[2-Acétylthio-3-(para-benzyloxycarbonyloxyphényl)propanoyl] glycine

2 g du composé obtenu dans la préparation D sont traités dans les conditions de la préparation B. Après chromatographie sur gel de silice dans le mélange hexane/acétate d'éthyle/acide acétique, 4:6:0,5, 1,35 g d'une huile qui cristallise lentement sont obtenus.
Rendement : 76 %
R_{f}: 0.25 (hexane/acétate d'éthyle/acide acétique, 4:6:0,5).

### PREPARATION F : Acide 2-acétylthio-3-(para-nitrophényl)propanoïque

A une solution de thioacétate de sodium (préparée à partir de 0,73 g d'hydrure de sodium et 1,05 ml d'acide thioacétique) dans le diméthylformamide sont ajoutés à 0°C et sous azote, 2,7 g d'acide 2-bromo-3-(*para*-nitrophényl)propanoïque (préparé à partir de la (*para*-nitrophényl)alanine). Après agitation 48 h à température ambiante, puis évaporation à sec et reprise du résidu à l'eau, le mélange est traité comme décrit dans la préparation C. 2,3 g d'une huile jaune pâle sont obtenus.
Rendement : 88%
R_{f} : 0,63 (dichlorométhane/méthanol/acide acétique, 9:1:0,2)

### PREPARATION G : Ester méthylique de la N-glycyl-5-(méta-hydroxyphényl)proline

### Etape a : Ester méthylique de la N-[(N-Cbz)-glycyl]-5-(méta-hydroxyphényl)proline

0,6 g de Z-glycine en solution dans du dichlorométhane sont couplés avec 0,83 g d'ester méthylique de la 5-(*méta*-hydroxyphényl)proline en présence de 2,06 g de PyBrOP et de 2,6 ml de diisopropyléthylamine. Après agitation 4 h à température ambiante, la phase organique est lavée (hydrogénocarbonate de sodium, eau, acide citrique, eau), séchée sur sulfate de sodium, filtrée et évaporée à sec. Le résidu huileux est chromatographié sur gel de silice (n-hexane/acétate d'éthyle, 4:6). Un solide blanc correspondant au produit attendu est obtenu.
Rendement : 33%
Point de fusion : 173°C
R_{f} : 0,17 (n-hexane/acétate d'éthyle, 4:6)

### Etape b : Ester méthylique de la N-glycyl-5-(méta-hydroxyphényl)proline

0,39 g du composé obtenu dans l'étape a sont hydrogénés à température et pression ordinaire en présence de palladium sur charbon 10% et de 1 équivalent d'acide chlorhydrique 1N en solution dans le méthanol. Après agitation 3 h à température ambiante, le catalyseur est filtré et le solvant évaporé à sec.
Rendement : 100%

### PREPARATION H : Ester méthylique de la N-[(N-Cbz)-glycyl]-5-(para-méthylphényl) proline

1 équivalent de Z-glycine en solution dans l'acétone est traité par le chlorure cyanurique en présence de triéthylamine. Après agitation 3 h à température ambiante, le mélange est évaporé à sec. Le résidu est repris dans du tétrachlorure de carbone et 1 équivalent de l'ester méthylique de la 5-(*para*-méthylphényl)proline est ajouté en solution dans du dichlorométhane et en présence de triéthylamine. Après 3 h d'agitation à température ambiante, le milieu réactionnel est évaporé à sec, repris par de l'acétate d'éthyle, lavé, séché, et à nouveau évaporé à sec. Le résidu huileux ainsi obtenu est chromatographié dans le mélange n-hexane/acétate d'éthyle, 1:1, pour fournir un solide blanc.
Rendement : 41%
R_{f} : 0,18 (n-hexane/acétate d'éthyle, 1:1)

### PREPARATION I : Ester méthylique de la N-[(N-Cbz)-glycyl]-5-(ortho-méthylphényl) proline

Ce composé est obtenu à partir de la Z-glycine et de l'ester méthylique de la *5-*(*ortho*-méthylphényl)proline selon le procédé décrit dans la préparation H. Le produit huileux ainsi obtenu est purifié par chromatographie dans le mélange n-hexane/acétate d'éthyle, 55:45. pour fournir une huile incolore.
Rendement : 42% R_{f}: 0,21 (n-hexane/acétate d'éthyle, 1:1)

### EXEMPLE 1 : Ester tertio-butylique de la N-[N-(2-acétylthio-3-phénylpropanoyl) glycyl]-5-phénylproline

A 6,1 g du composé obtenu dans la préparation B, en solution dans 40 ml de tétrahydrofurane, sont ajoutées successivement à 0°C, une solution de 5,4 g d'ester *tertio*-butylique de la 5-phénylproline et 3,35 ml de triéthylamine dans 40 ml de trichlorométhane, une solution de 2.96 g de 1-hydroxybenzotriazole dans 30 ml de tétrahydrofurane et une solution de 6,76 g de DCC dans 30 ml de trichlorométhane. La réaction est traitée comme décrit dans la préparation A. Le produit brut est purifié par chromatographie sur gel de silice avec le mélange hexane/acétate d'éthyle, 2:1 comme éluant. 6,47 g d'un composé huileux sont obtenus.
Rendement : 60%
R_{f} : 0,49 (acétate d'éthyle/hexane, 1:1)

### EXEMPLE 2 : N-[N-(2-Acétylthio-3-phénylpropanoyl)-glycyl]-5-phénylproline

A une solution de 3,2 g du composé obtenu dans l'exemple 1 dans 10 ml de dichlorométhane sont ajoutés, à 0°C, 5 ml d'acide trifluoroacétique. Le mélange est agité 30 min. à 0°C, puis 2 h 30 à température ambiante. Après évaporation à sec, le résidu huileux précipite dans un mélange éther/hexane, 1:1. 1,66 g d'un solide blanc sont obtenus.
Rendement : 58,5%
R_{f} : 0,57 (dichlorométhane/méthanol, 9:1)

### EXEMPLE 3 : N-[N-(2-Mercapto-3-phénylpropanoyl)glycyl]-5-phénylproline

0,700 g du composé obtenu dans l'exemple 2 sont dissous dans 10 ml de méthanol dégazé. 5 ml d'une solution 1M d'hydroxyde de sodium dégazé sont ajoutés à 0°C et le mélange est agité 1 heure à 0°C puis 3 h à température ambiante. Le milieu est acidifié par de l'acide chlorhydrique 1M jusqu'à pH 1 et est concentré sous vide. Le résidu est repris par de l'eau et la phase aqueuse extraite par de l'acétate d'éthyle. La phase organique est lavée à l'eau, par une solution saturée de chlorure de sodium, puis séchée sur sulfate de sodium. Après filtration et évaporation à sec, 610 mg d'une huile jaune pâle qui cristallise lentement sont obtenus.
Rendement : 96%
Point de fusion : 75°C
R_{f} : 0,24 (dichlorométhane/méthanol, 95:5)

### EXEMPLE 4 : Ester méthylique de la N-[N-(2-acétylthio-3-phénylpropanoyl) glycyl]-5-(ortho-hydroxyphényl)proline

0,500 g du composé obtenu dans la préparation B et 0,400 g d'ester méthylique de la 5-(*ortho*-hydroxyphényl)proline sont condensés dans les conditions de l'exemple 1. Après chromatographie sur gel de silice avec le mélange hexane/acétate d'éthyle, 6:4, 0,32 g du produit attendu sous forme d'une huile sont obtenus.
Rendement : 50%
R_{f} : 0,2 (cyclohexane/acétate d'éthyle, 1:1)

### EXEMPLE 5 : N-[N-(2-Mercapto-3-phénylpropanoyl)glycyl]-5-(ortho-hydroxyphényl) proline

0.300 g du composé obtenu dans l'exemple 4 sont traités comme décrits dans l'exemple 3. 0,200 g du composé attendu sont obtenus.
Rendement : 88%
R_{f} : 0,69 (dichlorométhane/méthanol/acide acétique, 9:1:0,5)

### EXEMPLE 6 : Ester méthylique de la N-[N-[2-acétylthio-3-(para-benzyloxy-carbonyloxyphényl)propanoyl]glycyl]-5-(ortho-hydroxyphényl)proline

A une solution de 0,500 g du composé obtenu dans la préparation E dans le dichlorométhane, sont ajoutés, à 0°C, 0,65 g d'ester méthylique de la 5-(*ortho*-hydroxyphényl)proline, 0,7 ml de diisopropyléthylamine et 1,27 g de BOP. L'agitation est maintenue à température ambiante pendant 48 h. Après traitement selon les conditions classiques, l'huile obtenue est purifiée par chromatographie dans le mélange hexane/acétate d'éthyle, 4:6. 0,35 g d'un produit huileux sont obtenus.
Rendement : 47%
R_{f} : 0,53 (cyclohexane/acétate d'éthyle/acide acétique, 3:7:0,2)

### EXEMPLE 7 : N-[N-[2-Mercapto-3-(para-hydroxyphényl)propanoyl]glycyl]-5-(ortho-hydroxyphényl)proline

A 0,200 g du composé obtenu dans l'exemple 6, dans 1 ml de méthanol dégazé, sont ajoutés, à 0°C, 2 ml d'hydroxyde de sodium 1M dégazé. Après agitation pendant 1 h à 0°C puis 2h à température ambiante, la réaction est traitée comme décrit dans l'exemple 3. 0,125 g d'un solide blanc sont obtenus.
Rendement : 89%
R_{f}: 0,34 (dichlorométhane/méthanol/acide acétique, 9:1:0,2)

### EXEMPLE 8 : Ester tertio-butylique de la N-[N-[2-acétylthio-3-(para-benzyloxy-carbonyloxyphényl)propanoyl]glycyl]-5-phénylproline

0.590 g du composé obtenu dans la préparation C sont condensés avec l'ester *tertio*-butylique du dipeptide Gly-(5-phényl)-Pro dans les conditions de la préparation A. 1 g du composé attendu est obtenu.
Rendement : 98%
R_{f} : 0.46 (hexane/acétate d'éthyle, 1:2)

### EXEMPLE 9 : N-[N-[2-Acétylthio-3-(para-hydroxyphényl)propanoyl]glycyl]-5-phénylproline

1 g du composé du composé obtenu dans l'exemple 8 est dissous dans un mélange de tris(trifluoroacétate) de bore et d'acide trifluoroacétique sous azote à 0°C. Le mélange est agité à la même température pendant 20 min. Après évaporation à sec, le résidu est repris 3 fois dans le méthanol et évaporé à nouveau à sec. Le résidu huileux précipite alors dans l'éther éthylique conduisant ainsi à 710 mg d'une poudre jaune pâle.
Rendement : 75%
R_{f} : 0,71 (dichlorométhane/méthanol/acide acétique, 9:1:0,5)

### EXEMPLE 10 : N-[N-[2-Mercapto-3-(para-hydroxyphényl)propanoyl]glycyl]-5-phénylproline

450 mg du composé obtenu dans l'exemple 9 sont dissous dans 5 ml de méthanol dégazé. 2 ml d'hydroxyde de sodium 1M sont ajoutés à 0°C, et la réaction est poursuivie comme décrit dans l'exemple 3. 400 mg du composé attendu sous forme d'un solide blanc sont obtenus.
Rendement : 97%
R_{f} : 0,83 (trichlorométhane/méthanol/acide acétique, 7:3:0,5)

### EXEMPLE 11 : Ester tertio-butylique de la N-[N-[2-acétylthio-3-(para-nitrophényl) propanoyl]glycyl]-5-phénylproline

Par réaction entre 1 g du composé obtenu dans la préparation F et 1,15 g d'ester *tertio*-butylique de la N-glycyl-5-phénylproline dans les conditions décrites dans l'exemple 1, est obtenu un composé huileux qui est purifié par chromatographie sur gel de silice dans le mélange cyclohexane/acétate d'éthyle, 1:1. 1,09 g d'une huile jaune pâle sont obtenus.
Rendement : 53%

### EXEMPLE 12 : N-[N-[2-Acétylthio-3-(para-nitrophényl)propanoyl]glycyl]-5-phénylproline

A 0.72 g du composé obtenu dans l'exemple 11, dissous dans 2 ml de dichlorométhane, sont ajoutés à 0°C, 2 ml d'acide trifluoroacétique. Après traitement dans les conditions décrites dans l'exemple 2, 0,58 g d'un solide blanc sont obtenus.
Rendement : 90%
R_{f} : 0,39 (dichlorométhane/méthanol, 9:1)

### EXEMPLE 13 : N-[N-[2-Acétylthio-3-(para-aminophényl)propanoyl]glycyl]-5-phénylproline

300 mg du composé obtenu dans l'exemple 12 sont mis en solution dans 15 ml de méthanol et quelques gouttes d'acide acétique, puis sont hydrogénés sous pression ordinaire en présence de palladium sur charbon. Après filtration du catalyseur et évaporation du solvant, 0,280 g d'un solide blanc sont obtenus.
Rendement : 100%
R_{f} : 0,31 (dichlorométhane/méthanol/acide acétique, 9:1:0,25)

### EXEMPLE 14 : N-[N-[2-Mercapto-3-(para-aminophényl)propanoyl]glycyl]-5-phénylproline

0,150 g du composé obtenu dans l'exemple 13 sont traités dans les conditions de l'exemple 3. Après traitement habituel de la phase organique, 0,103 g d'un solide blanc sont obtenus.
Rendement : 89%
R_{f} : 0.36 (dichlorométhane/méthanol/acide acétique, 9:1:0,3)

### EXEMPLE 15 : Ester méthylique de la N-[N-[2-acétylthio-3-(para-benzyloxy-carbonyloxyphényl)propanoyl]glycyl]-5-(para-aminophényl)proline

Le composé de la préparation E est couplé avec l'ester méthylique de la 5-(*para*-aminophényl)proline selon les conditions décrites dans l'exemple 6. On obtient un produit huileux qui est chromatographié dans un mélange dichlorométhane/méthanol/acide acétique, 15:0,4:0,1. Un produit mousseux blanc est obtenu.
Rendement : 35%
R_{f} : 0.25 (dichlorométhane/méthanol/acide acétique, 9:1:0,25)

### EXEMPLE 16 : N-[N'-[2-Mercapto-3-(para-hydroxyphényl)propanoyl]glycyl]-5-(para-aminophényl)proline.

Le composé obtenu dans l'exemple 15 est traité selon les conditions de l'exemple 7. Un solide blanc correspondant au produit attendu est obtenu.
Rendement : 60%
R_{f} : 0,37 (dichlorométhane/méthanol/acide acétique, 9:1:0,5)

### EXEMPLE 17 : Ester méthylique de la N-[N-[2-acétylthio-3-(para-benzyloxy-carbonyloxyphényl)propanoyl]glycyl]-5-(méta-hydroxyphényl)proline

0,35 g du composé obtenu dans la préparation C sont couplés avec 0,3 g du composé obtenu dans la préparation G selon le procédé décrit dans la préparation A. On obtient un produit huileux qui est chromatographié sur gel de silice dans un mélange hexane/acétate d'éthyle,
4:6. 0,24 g du produit attendu sont obtenus.
Rendement : 40%
R_{f} : 0,37 (n-hexane/acétate d'éthyle, 4:6)

### EXEMPLE 18 : N-[N-[2-Mercapto-3-(para-hydroxyphényl)propanoyl]glycyl]-5-(méta-hydroxyphényl)proline

0,24 g du composé obtenu dans l'exemple 17 sont dissous dans du méthanol dégazé puis traités par 4 equivalents d'une solution aqueuse dégazée d'hydroxyde de sodium 1N. Après agitation 30 min. à 0°C et 2 h à température ambiante, le milieu réactionnel est acidifié et extrait par l'acétate d'éthyle. Après évaporation à sec, 0,12 g d'un solide blanc sont obtenus.
Rendement : 71%
R_{f} : 0,34 (dichlorométhane/méthanol/acide acétique, 9:1:0,5)

### EXEMPLE 19 : Ester méthylique de la N-[N-(2-acétylthio-3-phénylpropanoyl)glycyl]-5-(méta-hydroxyphényl)proline

Le composé obtenu dans la préparation G est couplé avec l'acide 2-acétylthio-3-phénylpropanoïque selon le procédé décrit dans la préparation A. On obtient un produit huileux qui est chromatographié dans un mélange n-hexane/acétate d'éthyle, 1:1.
Rendement : 50%
R_{f} : 0.28 (n-hexane/acétate d'éthyle, 4:6)

### EXEMPLE 20 : N-[N-(2-Mercapto-3-phénylpropanoyl)glycyl]-5-(méta-hydroxyphényl) proline

Le composé obtenu dans l'exemple 19 est traité selon le procédé de l'exemple 3. Un produit pâteux correspondant au composé attendu est obtenu.
Rendement : 85%
R_{f} : 0.29 (dichlorométhane/méthanol/acide acétique, 9:0,5:0,25)

### EXEMPLE 21 : Ester méthylique de la N-[N-[2-acétylthio-3-(para-benzyloxy-carbonyloxyphényl)propanoyl] glycyl]-5-(para-hydroxyphényl)proline

Le composé obtenu dans la préparation C est couplé avec l'ester méthylique de la N-glycyl-5-(*para*-hydroxyphényl)proline, préparé selon les procédés décrits dans les étapes a et b de la préparation G. Un produit huileux correspondant au composé attendu est obtenu.
Rendement : 50%
R_{f} : 0,26 (cyclohexane/acétate d'éthyle/acide acétique, 5,5:4,5:0,5)

### EXEMPLE 22 : N-[N-[2-Mercapto-3-(para-hydroxyphényl)propanoyl]glycyl]-5-(para-hydroxyphényl)proline

Le composé dans l'exemple 21 est traité selon le procédé décrit dans l'exemple 3. On obtient un produit pâteux.
Rendement : 77%
R_{f} : 0,24 (dichlorométhane/méthanol/acide acétique, 9:1:0,5)

### EXEMPLE 23 : Ester méthylique de la N-[N-[2-acétylthio-3-(para-chlorophényl)-propanoyl]glycyl]-5-(ortho-hydroxyphényl)proline

La condensation de l'acide 2-acétylthio-3-(*para*-chlorophényl)propanoïque avec l'ester méthylique de la N-glycyl-5-(*ortho*-hydroxyphényl)proline, obtenue à partir des mêmes protocoles que ceux décrits dans la préparation G, conduit, après chromatographie dans le mélange n-hexane/acétate d'éthyle, 1:1, au produit attendu sous forme d'huile.
Rendement : 53%
R_{f}: 0.38 (n-hexane/acétate d'éthyle, 4:6)

### EXEMPLE 24 : N-[N-[2-Mercapto-3-(para-chlorophényl)propanoyl]glycyl]-5-(ortho-hydroxyphényl)proline

Le composé obtenu dans l'exemple 23 est traité selon le procédé décrit dans l'exemple 3. On obtient un solide blanc pâteux.
Rendement : 88%
R_{f} : 0,26 (dichlorométhane/méthanol/acide acétique, 9:0,5:0,25)

### EXEMPLE 25 : Ester méthylique de la N-[N-[2-acétylthio-3-(para-méthoxyphényl)-propanoyl]glycyl]-5-(ortho-hydroxyphényl)proline

La condensation de l'acide 2-acétylthio-3-(*para*-méthoxyphényl)propanoïque avec l'ester méthylique de la N-glycyl-5-(*ortho*-hydroxyphényl)proline, obtenue à partir des mêmes protocoles que ceux décrits dans la prépapration G, conduit, après chromatographie dans un mélange n-hexane/acétate d'éthyle, 4:6, au produit attendu sous forme d'huile.
Rendement : 50%
R_{f} : 0,28 (n-hexane/acétate d'éthyle, 4:6)

### EXEMPLE 26 : N-[N-[2-Mercapto-3-(para-méthoxyphényl)propanoyl]glycyl]-5-(ortho-hydroxyphényl)proline

Par traitement du composé obtenu dans l'exemple 25 selon les conditions décrites dans l'exemple 3, on obtient un solide blanc.
Rendement : 88%
R_{f} : 0,42 (dichlorométhane/méthanol/acide acétique, 9:0,5:0,25)

### EXEMPLE 27 : Ester méthylique de la N-[N-[2-acétylthio-3-(para-méthoxyphényl)-propanoyl]glycyl]-5-(ortho-méthoxyphényl)proline

La condensation de l'acide 2-acétylthio-3-(*para*-méthoxyphényl)propanoïque avec l'ester méthylique de la la N-glycyl-5-(*ortho*-méthoxyphényl)proline, obtenue à partir des mêmes protocoles que ceux décrits dans la préparation G, conduit après chromatographie dans le mélange n-hexane/acétate d'éthyle, 1:1, au produit attendu sous forme d'un solide blanc. Rendement : 59%
R_{f}: 0,26 (n-hexane/acétate d'éthyle, 4:6)

### EXEMPLE 28 : N-[N-[2-Mercapto-3-(para-méthoxyphényl)propanoyl]glycyl]-5-(ortho-méthoxyphényl)proline

Le traitement du composé obtenu dans l'exemple 27, selon les conditions de l'exemple 3, conduit à un solide blanc.
Rendement : 89%
R_{f} : 0,22 (cyclohexane/acétate d'éthyle/acide acétique, 5:5:0,5)

### EXEMPLE 29 : Ester méthylique de la N-[N-(2-acétylthio-3-phénylpropanoyl) glycyl]-5-(para-méthylphényl)proline

Le composé obtenu dans la préparation H est déprotégé selon le procédé décrit dans l'étape b de la préparation G ; le dipeptide obtenu est couplé avec l'acide 2-acétylthio-3-phénylpropanoïque selon le procédé décrit dans la préparation A. Le produit huileux ainsi obtenu est chromatographié dans un mélange n-hexane/acétate d'éthyle, 5,5:4,5, pour fournir un solide blanc.
Rendement : 69%
R_{f} : 0,55 (n-hexane/acétate d'éthyle, 4:6)

### EXEMPLE 30 : N-[N-(2-Mercapto-3-phénylpropanoyl)glycyl]-5-(para-méthylphényl) proline

Le composé obtenu dans l'exemple 29 est traité selon le procédé décrit dans l'exemple 3. On obtient un solide blanc.
Rendement : 89%
R_{f} : 0.59 (dichlorométhane/méthanol/acide acétique, 9:0,5:0,5)

### EXEMPLE 31 : Ester méthylique de la N-[N-[2-acétylthio-3-(para-hydroxyphényl)-propanoyl]glycyl]-5-(para-méthylphényl)proline

Le composé obtenu dans la préparation H est déprotégé selon le procédé décrit dans l'étape b de la préparation G ; le dipeptide obtenu est couplé avec le composé obtenu dans la préparation C selon le procédé décrit dans la préparation A. Le produit huileux ainsi obtenu est chromatographié dans un mélange n-hexane/acétate d'éthyle, 1:1, pour fournir un solide blanc.
Rendement : 52%
R_{f}: 0.14 (n-hexane/acétate d'éthyle, 1:1)

### EXEMPLE 32 : N-[N-[2-Mercapto-3-(para-hydroxyphényl)propanoyl]glycyl]-5-(para-méthylphényl)proline

Le composé obtenu dans l'exemple 31 est traité selon le procédé décrit dans l'exemple 3. On obtient un solide blanc.
Rendement : 92%
R_{f} : 0,60 (dichlorométhane/méthanol/acide acétique, 9:1:0,5)

### EXEMPLE 33 : Ester méthylique de la N-[N-[2-acétylthio-3-(para-hydroxyphényl) propanoyl]glycyl]-5-(ortho-méthylphényl)proline

Le composé obtenu dans la préparation I est déprotégé selon le procédé décrit dans l'étape b de la préparation G et le dipeptide obtenu est couplé avec le composé obtenu dans la préparation C selon le procédé décrit dans la préparation A. Le produit huileux ainsi obtenu est purifié par chromatographie dans le mélange n-hexane/acétate d'éthyle, 1:1 pour fournir une huile.
Rendement : 56%
R_{f} : 0,28 (n-hexane/acétate d'éthyle, 4:6)

### EXEMPLE 34 : N-[N-[2-Mercapto-3-(para-hydroxyphényl)propanoyl]glycyl]-5-(ortho-méthylphényl)proline

Le composé obtenu dans l'exemple 33 est traité selon le procédé de l'exemple 3. On obtient un solide blanc.
Rendement : 87%
Point de fusion : 130°C (décomposition)
R_{f} : 0,24 (n-hexane/acétate d'éthyle, 4:6)

### EXEMPLE 35 : Ester méthylique de la N-[N-(2-acétylthio-3-phénylpropanoyl)glycyl]-5-(ortho-méthylphényl)proline

Le composé obtenu dans la préparation I est déprotégé selon le procédé décrit dans l'étape b de la préparation G et le dipeptide obtenu est couplé avec l'acide 2-acétylthio-3-phénylpropanoïque selon le procédé de la préparation A. Le solide blanc ainsi obtenu est chromatographié dans un mélange n-hexane/acétate d'éthyle, 55:45.
Rendement : 58%
Point de fusion : 97°C
R_{f}: 0.40 (n-hexane/acétate d'éthyle, 4:6)

### EXEMPLE 36 : N-[N-(2-Mercapto-3-phénylpropanoyl)glycyl]-5-(ortho-méthylphényl)-proline

Le composé obtenu dans l'exemple 35 est traité selon le procédé de l'exemple 3. On obtient un solide blanc.
Rendement : 63%
Point de fusion : 110°C (décomposition)
R_{f}: 0.15 (dichlorométhane/méthanol/acide acétique, 9:0,7:0,25)

En utlisant des procédés analogues, les composés des exemples suivants sont obtenus :

### EXEMPLE 37 : N-[N-(2-Propanoylthio-3-phénylpropanoyl)glycyl]-5-(ortho-hydroxyphényl)proline

### EXEMPLE 38 : N-[N-(2-Benzoylthio-3-phénylpropanoyl)glycyl]-5-phénylproline

### EXEMPLE 39 : N-[N-[2-Mercapto-3-(para-sulfamoylphényl)propanoyl]glycyl]-5-phénylproline

### EXEMPLE 40 : N-[N-[2-Mercapto-3-(para-propylsulfamoylphényl)propanoyl]glycyl]-5-(méta-hydroxyphényl)proline

### EXEMPLE 41 : Ester cyclohexylméthylique de la N-[N-[2-acétylthlo-3-(para-sulfamoyl-phényl)propanoyl]glycyl)-5-(méta-hydroxyphényl)proline

### EXEMPLE 42 : Ester benzylique de la N-[N-[2-mercapto-3-(para-hydroxyphényl) propanoyl]glycyl]-5-(méta-hydroxyphényl)proline

### EXEMPLE 43 : Acide N-[N-[2-mercapto-3-(para-chlorophényl)propanoyl]glycyl]-2-(ortho-hydroxyphényl)imidazolidine-5-carboxylique

### EXEMPLE 44 : Acide N-[N-(2-mercapto-3-phénylpropanoyl)glycyl]-2-phényloxazolidine-5-carboxylique

### EXEMPLE 45 : Acide N-[N-(2-mercapto-3-phénylpropanoyl)glycyl]-2-(ortho-hydroxyphényl)thiazolidine-5-carboxylique

### EXEMPLE 46 : Acide N-[N-[2-mercapto-3-(para-hydroxyphényl)propanoyl]glycyl]-2-(méta-hydroxyphényl)hexahydropyrimidinyl-6-carboxylique

### EXEMPLE 47 : N-[N-(5R)-[(2S)-2-mercapto-3-(para-hydroxyphényl)propanoyl]glycyl]-5-(méta-hydroxyphényl)-L-proline

La (5*R*)-5-(*méta*-hydroxyphényl)-L-proline, obtenue selon le mode opératoire décrit par J. Ezquerra et al. (*Tetrahedron Letters, 34* (39), (1993), 6317-6320), est traitée dans les conditions décrites à la préparation G, puis couplée, selon le mode opératoire décrit à la préparation A, avec l'acide (2*S*)-2-acétylthio-3-(*para*-benzyloxycarbonyloxyphényl)-propanoïque obtenu à la préparation C. L'ester ainsi obtenu est saponifié comme décrit à l'exemple 18 pour obtenir le produit titre attendu.

Les composés des exemples 48 à 53 sont obtenus comme décrit dans l'exemple 47.

### EXEMPLE 48 : N-[N-(5R)-[(2S)-(2-mercapto-3-phénylpropanoyl)glycyl]-5-phényl]-L-proline

### EXEMPLE 49 : N-[N-(5R)-[(2S)-(2-mercapto-3-phénylpropanoyl)glycyl]-5-(ortho-hydroxyphényl)]-L-proline

### EXEMPLE 50 : N-[N-(5R)-[(2S)-[2-mercapto-3-(para-chlorophényl)propanoyl]glycyl]-5-(ortho-hydroxyphényl)-L-proline

### EXEMPLE 51 : N-(N-(5R)-[(2S)-[(2-mercapto-3-(para-aminophényl)propanoyl]glycyl]-5-phényl]-L-proline

### EXEMPLE 52 : N-[N-(5S)-[(2S)-2-mercapto-3-(para-hydroxyphényl)propanoyl]glycyl]-5-(méta-hydroxyphényl]-D-proline

### EXEMPLE 53 : N-[N-(5R)-[(2R)-2-mercapto-3-(para-hydroxyphényl)propanoyl]glycyl]-5-(méta-hydroxyphényl]-L-proline

### EXEMPLE 54 : N-[N-(5S)-[(2S)-2-mercapto-3-(para-hydroxyphényl)propanoyl]glycyl]-5-(méta-hydroxyphényl]-L-proline

### Etape a : Acide (2S)-5-(méta-hydroxyphényl)-3,4-dihydro[2H]pyrrole-2-carboxylique

Composé obtenu selon le protocole décrit par Gershon et al. (*J*. *Med. Chem.,* (1965), *8*, 877-881).

### Etape b : (5S)-5-(méta-hydroxyphényl)-L-proline

Le composé obtenu à l'étape a est réduit (traitement par OH⁻, H⁺, puis NaBH₄) pour fournir un mélange d'isomères (2*S*, 5*R*) et (2*S*, 5*S*) qui sont séparés selon le mode opératoire décrit par C. G. Overberger et al. (*Macromolecules*, (1972), *5* (4), 368-372).

### Etape c : N-[N-(5S)-[(2S)-2-mercapto-3-(para-hydroxyphényl)propanoyl]glycyl]-5-(méta-hydroxyphényl]-L-proline

Le composé titre est obtenu à partir du produit obtenu à l'étape précédente selon le mode opératoire décrit à l'exemple 47.

### EXEMPLE 55 : N-[N-(5R)-[(2S)-2-mercapto-3-(para-hydroxyphényl)propanoyl]glycyl]-5-(méta-hydroxyphényl]-D-proline

Composé obtenu selon le mode opératoire décrit à l'exemple 54, à partir de l'acide (2*R*)-5-(*méta*-hydroxyphényl)-3,4-dihydro[2*H*]pyrrole-2-carboxylique.

### ETUDE PHARMACOLOGIQUE

### EXEMPLE A : Effet inhibiteur in vitro sur l'endopeptidase neutre (NEP) et la peptidyldipeptidase A (ACE)

Les pouvoirs inhibiteurs des composés de l'invention sont mesurés en utilisant la [³H]-D-Ala²-Leu-enképhaline comme substrat pour l'endopeptidase neutre, selon le protocole décrit par Llorens *et al.* (*Biochem. Biophys. Res. Commun.,* ***96,*** (1980), 1710 sqq) et la Z-Phe-His-Leu comme substrat dans le cas de la peptidyldipeptidase A selon le protocole décrit dans *Biochem*. *Biophys*. *Acta*, ***206,*** (1970), 136-142.

Les résultats sont rassemblés dans le tableau I dans lequel sont également donnés les résultats obtenus avec la N-(2-mercaptométhyl-3-phényl-1-oxopropyl)glycine (thiorphan) qui, bien que de structure très voisine, manifeste uniquement une activité sur l'endopeptidase neutre (NEP).

**Tableau I**

| Effet inhibiteur sur l'endopeptidase neutre (NEP) et l'enzyme de conversion de l'angiotensine (ACE) | | |
|---|---|---|
| ***COMPOSE*** | ***IC***_{***50***}***(nM) NEP*** | ***IC***_{***50***}***(nM) ACE*** |
| Exemple 3 | 1,6 ± 0,3 | 0,55 ± 0,05 |
| Exemple 5 | 1,1 ± 0,3 | 0,50 ± 0,10 |
| Exemple 7 | 4,3 ± 0,3 | 0,35 ± 0,05 |
| Exemple 10 | 3,4 ± 0,8 | 4,2 ± 0,4 |
| Exemple 14 | 1,8 ± 0,4 | 3,5 ± 0,5 |
| Exemple 16 | 26 ± 3 | 23 ± 3 |
| Exemple 18 | 1,6 ± 0,3 | 0,7 ± 0,2 |
| Exemple 20 | 2,4 ± 0,8 | 1,4 ± 0,7 |
| Exemple 22 | 6 ± 1 | 0,45 ± 0,05 |
| Exemple 24 | 1,2 ± 0,4 | 0,90 ± 0,06 |
| Exemple 26 | 2,3 ± 0,3 | 0,40 ± 0,05 |
| Exemple 28 | 2,5 ± 0,6 | 2,20 ± 0,20 |
| Exemple 30 | 10 ± 2 | 8,2 ± 0,5 |
| Exemple 32 | 5,0 ± 0,5 | 3,2 ± 0,4 |
| Exemple 34 | 3,1 ± 0,3 | 5,2 ± 0,5 |
| Exemple 36 | 4,0 ± 0,6 | 3,5 ± 0,6 |
| Thiorphan | 2,0 ± 0,4 | 140 ± 13 |

### EXEMPLE B : Effet inhibiteur in vivo sur l'endopeptidase neutre rénale (NEP) et la peptidyldipeptidase A pulmonaire (ACE)

Les composés sont administrés par voie orale chez la souris à une dose unique. L'inhibition des deux enzymes est mesurée sur une période de temps qui va de 1 h à 16 h par des expériences de compétition avec des marqueurs spécifiques radiomarqués de chaque enzyme.

Après administration par voie i.v. d'un inhibiteur d'ACE radiomarqué ([³H]trandolaprilate) ou d'un inhibiteur de NEP radiomarqué ([³H]HACBOGly), l'animal est sacrifié et les poumons ou les reins sont prélevés. Les organes sont homogénéisés et la radioactivité associée à ces homogénats est mesurée par scintillation liquide comme décrit dans le protocole de M.C. Fournie-Zaluski et coll. (*J*. *Med. Chem., 37*, (1994), 1070-1083).

A titre d'exemple, le composé décrit dans l'exemple 47 a fourni les résultats présentés dans le tableau II suivant :

**Tableau II**

| Inhibition *in vivo* de la NEP rénale et de l'ACE pulmonaire (Composé de l'Exemple 47 : dose administrée *p.o.* : 2,6.10⁻⁶ mol/kg soit 11,5 mg/kg) | | |
|---|---|---|
| ***temps écoulé après administration*** | ***% d'inhibition*** | |
| | ***NEP rénale*** | ***ACE pulmonaire*** |
| 1 h | 75 ± 3 | 90 ± 1 |
| 2 h | 70 ± 5 | 86 ± 3 |
| 4 h | 74 ± 4 | 87 ± 3 |
| 8 h | 60 ± 10 | 73 ± 3 |
| 16 h | 48 ± 4 | 20 ± 8 |

### EXEMPLE C : Effets anti-hypertenseurs

L'effet anti-hypertenseur des composés de l'invention est déterminé *in vivo* chez le rat affecté d'une hypertension induite par l'administration de DOCA-sel (acétate de désoxy-corticostérone) et chez le rat mâle spontanément hypertendu (SHR), selon le protocole décrit par Trapani *et al.* (*J. Cardiovasc. Pharmacol*., ***14,*** (1989), 419-424). Dans ce test, les composés de l'invention se sont montrés être de puissants anti-hypertenseurs et ne présentent aucune toxicité.

A titre d'exemple, le composé décrit dans l'exemple 47 a fourni chez le rat SHR, les résultats présentés dans le tableau III suivant :

**Tableau III**

| Etude de l'effet anti-hypertenseur chez le rat SHR *(Administration p.o*., *une dose unique par jour de 25 mg/kg ; les animaux sont traités pendant 6 jours, première administration Jour 1*, *dernière administration Jour 6)* | | |
|---|---|---|
| Temps (jours) | Pression artérielle (mm Hg) | |
| | Rats témoins (n = 10) | Rats traités (n = 10) |
| 0 | 210 ± 5 | 210 ± 5 |
| 1 | 212 ± 3 | 195 ± 3* |
| 2 | 211 ± 1 | 189 ± 5* |
| 3 | 210 ± 2 | 183 ± 4* |
| 4 | 205 ± 2 | 184 ± 3* |
| 5 | 205 ± 1 | 182 ± 3* |
| 6 | 213 ± 2 | 185 ± 5* |
| 7 | 209 ± 4 | 189 ± 3 |
| 8 | 212 ± 2 | 198 ± 5 |
| 9 | 208 ± 2 | 203 ± 5 |
| 10 | 214 ± 1 | 210 ± 3 |

| | | |
|---|---|---|
| * p<0,01 ▭ traitement | | |

### EXEMPLE D : Composition pharmaceutique : Comprimés

Formule de préparation pour 1000 comprimés dosés à 10 mg

| | |
|---|---|
| Composé de l'exemple 5 | 10 g |
| Lactose | 50 g |
| Stéarate de magnésium | 10 g |
| Amidon de blé | 20 g |
| Amidon de maïs | 10 g |
| Silice | 5 g |
| Hydroxypropylcéllulose | 5 g |

## Revendications

1. Composés de formule (I): 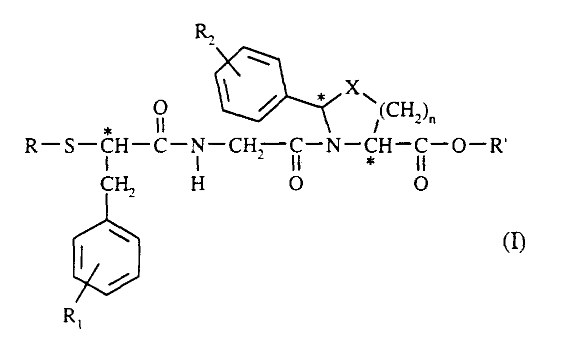 dans laquelle :
- **R** est choisi parmi l'hydrogène, un radical acyle contenant de 1 à 6 atomes de carbone en chaîne droite ou ramifiée, le radical benzoyle, naphtoyle, adamantoyle et le radical 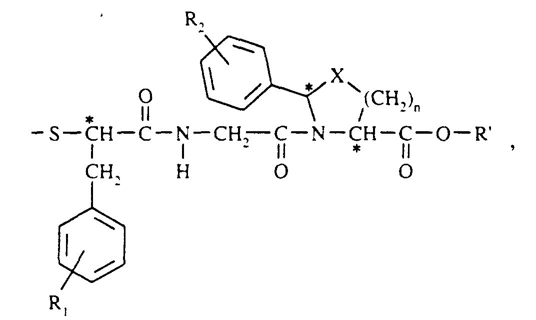 dans lequel R₁, R₂, R', X et n sont définis ci-après
- **R**_{**1**} et **R**_{**2**} identiques ou différents, sont choisis, indépendamment l'un de l'autre, parmi l'hydrogène, le radical hydroxy, un radical alkyle contenant de 1 à 6 atomes de carbone en chainc droite ou ramifiée, un radical alkoxy contenant de 1 à 6 atomes de carbone, en chaîne droite ou ramifiée, un atome d'halogène, choisi parmi fluor, chlore, brome et iode, un groupement nitro, un groupement amino, le radical carboxy, un radical alkoxycarbonyle contenant de 1 à 6 atomes de carbone en chaîne droite ou ramifiée, le radical benzyloxycarbonyloxy, le radical phosphonate, le radical sulfonate, le radical SO₂-NH₂ et le radical SO₂-NH-alkyle, dans lequel le terme alkyle désigne un groupement hydrocarboné saturé contenant de 1 à 6 atomes de carbone, en chaîne droite ou ramifiée,
- **R'** est choisi parmi l'hydrogène, un radical alkyle contenant de 1 à 20 atomes de carbone en chaîne droite ou ramifiée, un radical aralkyle et un radical cycloalkylalkyle,
- **X** est choisi parmi l'oxygène, le soufre, le groupement CH₂ et le groupement NH,
- **n** est choisi parmi les valeurs entières 1 et 2,
étant entendu que :
- "radical aralkyle" désigne un radical formé d'un groupement aryle, choisi parmi phényle et naphtyle, lié à un radical alkyle contenant de 1 à 6 atomes de carbone en chaîne droite ou ramifiée,
- "radical cycloalkylalkyle" désigne un radical formé d'un groupement cycloalkyle contenant de 3 à 8 atomes de carbone, lié à un radical alkyle contenant de 1 à 6 atomes de carbone en chaîne droite ou ramifiée,
ainsi que leurs formes stéréoisomériques à l'état pur ou en mélange et leurs éventuels sels d'addition, pharmaceutiquement acceptables.

2. Composés selon la revendication 1 pour lesquels n = 1 et X représente le groupement CH₂, leurs formes stéréoisomériques à l'état pur ou en mélange et leurs éventuels sels pharmaceutiquement acceptables.

3. Composés selon la revendication 1 dans lesquels :
- l'atome de carbone portant le radical 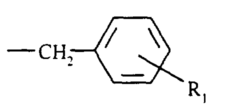 adopte la configuration S, et
- l'atome de carbone portant le radical 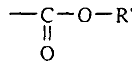 adopte la configuration S, et
- les radicaux 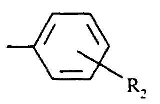 d'une part et 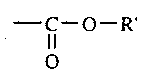 d'autre part sont disposés par rapport au plan moyen du cycle en configurations *cis,*
leurs formes stéréoisomériques à l'état pur ou en mélange et leurs éventuels sels pharmaceutiquement acceptables.

4. Composé selon la revendication 1 qui est la N-[N-(5*R*)-[(2*S*)-2-mercapto-3-(*para*-hydroxyphényl)propanoyl]glycyl]-5-(*méta*-hydroxyphényl)-L-proline.

5. Composé selon la revendication 1 qui est la N-[N-(5*R*)-[(2*S*)-(2-mercapto-3-phénylpropanoyl)glycyl]-5-phényl]-L-proline.

6. Composé selon la revendication 1 qui est la N-[N-(5*R*)-[(2*S*)-(2-mercapto-3-phénylpropanoyl)glycyl]-5-(*ortho*-hydroxyphényl)]-L-proline.

7. Composé selon la revendication 1 qui est la N-[N-(5*R*)-[(2*S*)-[2-mercapto-3-(*para*-chlorophényl)propanoyl]glycyl]-5-(*ortho*-hydroxyphényl)-L-proline.

8. Composé selon la revendication 1 qui est la N-[N-(5*R*)-[(2*S*)-[2-mercapto-3-(*para*- aminophényl)propanoyl]glycyl]-5-phényl]-L-proline.

9. Procédé de préparation des composés selon la revendication 1, caractérisé en ce que dipeptide de formule (II) : 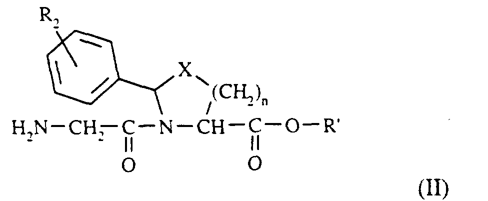 dans laquelle R₂, R', X et n sont tels que définis pour le composé de formule (I),
est soumis à une réaction d'acylation au moyen d'un acide de formule (III) : 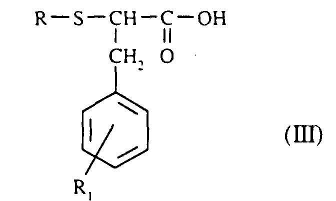 dans laquelle R et R₁ sont tels que définis pour le composé de formule (I),
dans les conditions habituelles utilisées en synthèse peptidique,
les dipeptides de formule (II) étant obtenus par condensation du chlorure d'acyle de la Z-glycine de formule (IV) : 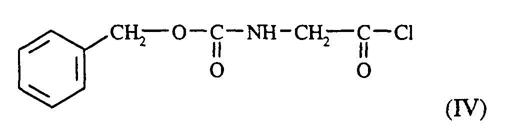 avec un ester de formule (V) : 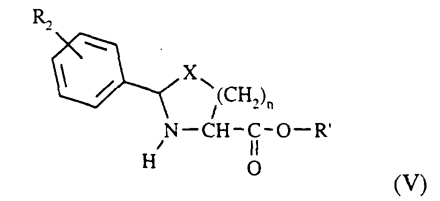 dans laquelle R₂, R', X et n sont tels que définis précédemment, suivie d'une hydrogénation catalytique.

10. Compositions pharmaceutiques contenant comme principe actif au moins un composé selon l'une quelconque des revendications 1 à 8, en combinaison avec un ou plusieurs véhicules inertes, non toxiques et pharmaceutiquement acceptables.

11. Compositions pharmaceutiques selon la revendication 10 exerçant une activité inhibitrice mixte de l'endopeptidase neutre (NEP) et de la peptidyldipeptidase A (ACE), utiles dans la prévention et le traitement des défaillances cardiaques congestives et des hypertensions essentielles, des hypertensions liées à une augmentation du volume sanguin, de l'athérosclérose, de l'ischémie cardiaque, de l'insuffisance cardiaque et/ou rénale, du coeur pulmonaire et de l'angine de poitrine, et utiles par leur effet protecteur sur l'épaississement de la paroi vasculaire, ainsi que par leur capacité à diminuer les effets secondaires dus aux traitements par la cyclosporine.

## Patentansprüche

1. Verbindungen der Formel (I): 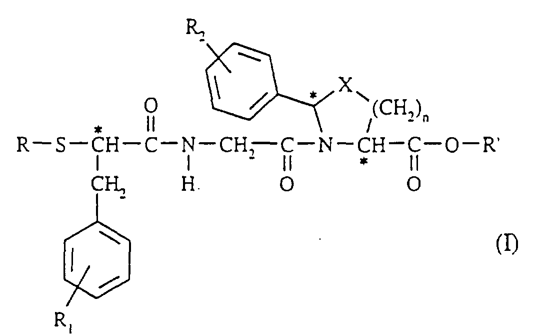 in der:
- **R** ausgewählt ist aus Wasserstoff, einer Acylgruppe, die 1 bis 6 Kohlenstoffatomen in gerader oder verzweigter Kette enthält, der Benzoyl-, Naphthoyl- und Adamantoylgruppe und der Gruppe der Formel 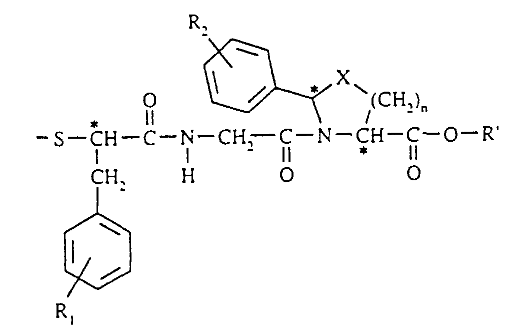 in der R₁, R₂, R', X und n nachfolgend definiert werden,
- **R**_{**1**} und **R**_{**2**}**,** die gleichartig oder verschieden sind, unabhängig voneinander ausgewählt sind aus Wasserstoff, der Hydroxygruppe, einer Alkylgruppe, die 1 bis 6 Kohlenstoffatome in gerader oder verzweigter Kette enthält, einer Alkoxygruppe, die 1 bis 6 Kohlenstoffatome in gerader oder verzweigter Kette enthält, einem Halogenatom, ausgewählt aus Fluor, Chlor, Brom und Iod, einer Nitrogruppe, einer Aminogruppe, der Carboxygruppe, einer Alkoxycarbonylgruppe, die 1 bis 6 Kohlenstoffatome in gerader oder verzweigter Kette enthält, der Benzyloxycarbonyloxygruppe, der Phosphonatgruppe, der Sulfonatgruppe, der Gruppe der Formel SO₂-NH₂ und der Gruppe SO₂-NH-Alkyl, worin der Begriff Alkyl für eine gesättigte Kohlenwasserstoffgruppe steht, die 1 bis 6 Kohlenstoffatome in gerader oder verzweigter Kette enthält,
- **R'** ausgewählt ist aus Wasserstoff, einer Alkylgruppe, die 1 bis 20 Kohlenstoffatome in gerader oder verzweigter Kette enthält, einer Aralkylgruppe und einer Cycloalkylalkylgruppe,
- **X** ausgewählt ist aus Sauerstoff, Schwefel, der CH₂-Gruppe und der NH-Gruppe,
- **n** ausgewählt ist aus den ganzen Werten 1 und 2,
mit der Maßgabe:
- daß die "Aralkylgruppe" für eine Gruppe steht, die aus einer Arylgruppe, ausgewählt aus Phenyl und Naphthyl, die an eine Alkylgruppe, die 1 bis 6 Kohlenstoffatome in gerader oder verzweigter Kette enthält, gebunden ist, gebildet ist,
- die "Cycloalkylalkylgruppe" für eine Gruppe steht, die aus einer Cycloalkylgruppe, die 3 bis 8 Kohlenstoffatome aufweist, und an eine Alkylgruppe, die 1 bis 6 Kohlenstoffatome in gerader oder verzweigter Kette aufweist, gebunden ist, gebildet ist,
sowie deren stereoisomere Formen in reinem Zustand oder in Form von Mischungen und gegebenenfalls ihre pharmazeutisch annehmbaren Additionssalze.

2. Verbindungen nach Anspruch 1, worin n = 1 und X die CH₂-Gruppe bedeuten, deren stereoisomere Formen in reinem Zustand oder in Form einer Mischung und deren eventuellen pharmazeutisch annehmbaren Salze.

3. Verbindungen nach Anspruch 1, worin:
- das die Gruppe 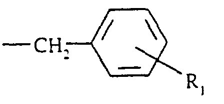 tragende Kohlenstoffatom in der Konfiguration S vorliegt und
- das die Gruppe 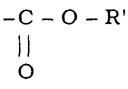 tragende Kohlenstoffatom in der Konfiguration S vorliegt, und
- die Gruppen 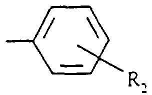 einerseits und 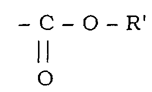 andererseits in bezug auf die Mittelebene des Rings in der *cis*-Konfiguration angeordnet sind,
deren stereoisomere Formen in reinem Zustand oder in Form einer Mischung und deren eventuelle pharmazeutisch annehmbaren Salze.

4. Verbindung nach Anspruch 1, nämlich N-[N-(5*R*)-[(2*S*)-2-Mercapto-3-(parahydroxyphenyl)-propanoyl]-glycyl]-5-(*meta*-hydroxyphenyl)-L-prolin.

5. Verbindung nach Anspruch 1, nämlich N-(N-(5*R*)-[(2*S*)-(2-Mercapto-3-phenylpropanoyl)-glycyl]-5-phenyl]-L-prolin.

6. Verbindung nach Anspruch 1, nämlich N-[N-(5*R*)-[(2*S*)-(2-Mercapto-3-phenylpropanoyl)-glycyl]-5-(*ortho*-hydroxyphenyl)-L-prolin.

7. Verbindung nach Anspruch 1, nämlich N-[N-(5*R*)-[(2*S*)-[2-Mercapto-3-(parachlorphenyl)-propanoyl]-glycyl]-5-(*ortho*-hydroxyphenyl)-L-prolin.

8. Verbindung nach Anspruch 1, nämlich N-[N-(5*R*)-[(2*S*)-[2-Mercapto-3-(*para*-aminophenyl)-propanoyl]-glycyl]-5-phenyl)-L-prolin.

9. Verfahren zur Herstellung der Verbindungen nach Anspruch 1, dadurch gekennzeichnet, daß man das Dipeptid der Formel (II): 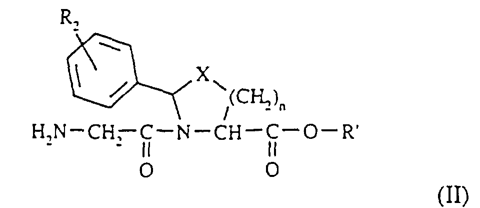 in der R₂, R', X und n die bezüglich der Verbindung der Formel (I) angegebenen Bedeutungen besitzen,
einer Acylierungsreaktion mit Hilfe einer Säure der Formel (III): 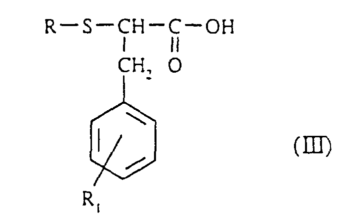 in der R und R1 die bezüglich der Verbindung der Formel (I) angegebenen Bedeutungen besitzen,
unter den üblicherweise in der Peptidsynthese angewandten Bedingungen unterwirft, wobei die Dipeptide der Formel (II) erhalten worden sind durch Kondensation des Acylchlorids des Z-Glycins der Formel (IV): 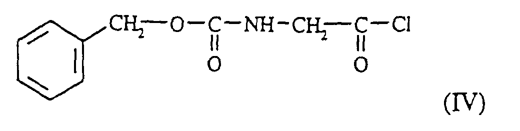 mit einem Ester der Formel (V): 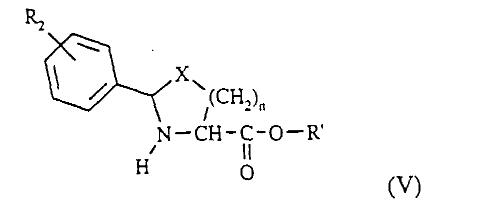 in der R₂, R', X und n die oben angegebenen Bedeutungen besitzen, gefolgt von einer katalytischen Hydrierung.

10. Pharmazeutische Zubereitungen enthaltend als Wirkstoff mindestens eine Verbindung nach einem der Ansprüche 1 bis 8 in Kombination mit einem oder mehreren inerten, nichttoxischen und pharmazeutisch annehmbaren Trägermaterialien.

11. Pharmazeutische Zubereitung nach Anspruch 10 mit einer gemischten inhibierenden Wirkung auf neutrale Endopeptidase (NEP) und Peptidyldipeptidase A (ACE) zur Vorbeugung und zur Behandlung von kongestiven Herzstörungen und essentiellen Hypertonien, Hypertonien, die mit einer Erhöhung des Blutvolumens verbunden sind, der Atherosklerose, der kardialen Ischämie, der Herz- und/oder Niereninsuffizienz, Lungenembolie und Angina pectoris und die aufgrund ihrer schützenden Wirkung auf die Verdickung der Gefäßwandungen sowie aufgrund ihrer Fähigkeit, die Nebenwirkungen der Behandlungen mit Cyclosporin zu verringern, nützlich sind.

## Claims

1. Compounds of formula (I) : 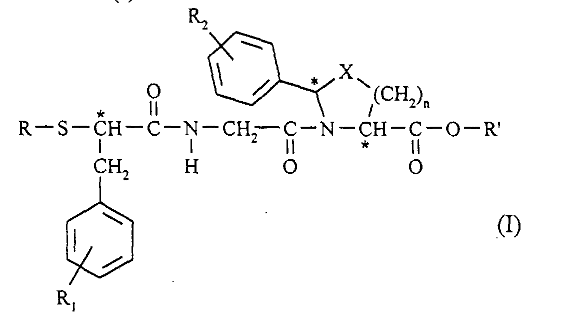 wherein :
- **R** is selected from hydrogen, an acyl radical containing from 1 to 6 carbon atoms in straight or branched chain, the radicals benzoyl, naphthoyl and adamantoyl and the radical 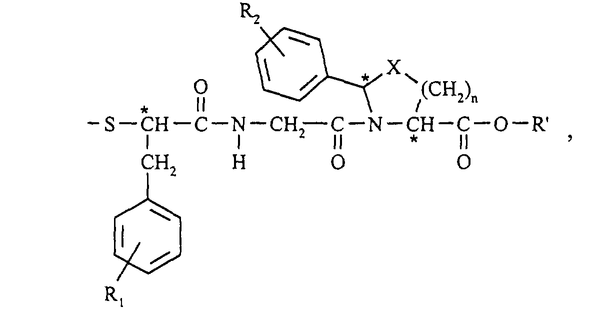 wherein R₁, R₂, R', X and n are as defined below,
- **R**_{**1**} and **R**_{**2**}**,** which are identical or different, are each selected, independently of the other, from hydrogen, a hydroxy radical, an alkyl radical containing from 1 to 6 carbon atoms in straight or branched chain, an alkoxy radical containing from 1 to 6 carbon atoms in straight or branched chain, a halogen atom selected from fluorine, chlorine, bromine and iodine, a nitro group, an amino group, a carboxy radical, an alkoxycarbonyl radical containing from 1 to 6 carbon atoms in straight or branched chain, a benzyloxycarbonyloxy radical, a phosphonate radical, a sulphonate radical, the radical SO₂-NH₂ and the radical SO₂-NH-alkyl wherein the term alkyl denotes a saturated hydrocarbon group containing from 1 to 6 carbon atoms in straight or branched chain,
- **R'** is selected from hydrogen, an alkyl radical containing from 1 to 20 carbon atoms in straight or branched chain, an aralkyl radical and a cycloalkylalkyl radical,
- **X** is selected from oxygen, sulphur, the group CH₂ and the group NH,
- **n** is selected from the integers 1 and 2,
it being understood that :
- "aralkyl radical " denotes a radical formed by an aryl group selected from phenyl and naphthyl that is bonded to an alkyl radical containing from 1 to 6 carbon atoms in straight or branched chain,
- "cycloalkylalkyl radical" denotes a radical formed by a cycloalkyl group containing from 3 to 8 carbon atoms that is bonded to an alkyl radical containing from 1 to 6 carbon atoms in straight or branched chain,
and also their stereoisomeric forms in pure form or in a mixture, and their possible pharmaceutically acceptable addition salts.

2. Compounds according to claim 1 wherein n = 1 and X represents the group CH₂, their stereoisomeric forms in pure form or in a mixture, and their possible pharmaceutically acceptable salts.

3. Compounds according to claim 1 wherein :
- the carbon atom carrying the radical 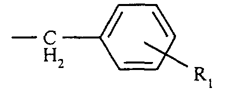 has the S configuration, and
- the carbon atom carrying the radical 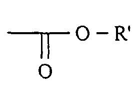 has the S configuration, and
- the radicals 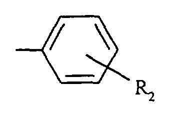 on the one side and 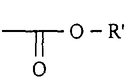 on the other side are located in the *cis* configuration in relation to the mean plane of the ring,
their stereoisomeric forms in pure form or in a mixture, and their possible pharmaceutically acceptable salts.

4. Compound according to claim 1 which is N-[N-(5*R*)-[(2*S*)-2-mercapto-3-(*para*-hydroxyphenyl)propanoyl]glycyl]-5-(*meta*-hydroxyphenyl)-L-proline.

5. Compound according to claim 1 which is N-[N-(5*R*)-[(2*S*)-(2-mercapto-3-phenyl-propanoyl)glycyl]-5-phenyl]-L-proline.

6. Compound according to claim 1 which is N-[N-(5*R*)-[(2*S*)-(2-mercapto-3-phenyl-propanoyl)glycyl]-5-(*ortho*-hydroxyphenyl)]-L-proline.

7. Compound according to claim 1 which is N-[N-(5*R*)-[(2*S*)-[2-mercapto-3-(*para*-chlorophenyl)propanoyl]glycyl]-5-(*ortho*-hydroxyphenyl)]-L-proline.

8. Compound according to claim 1 which is N-[N-(5*R*)-[(2*S*)-[2-mercapto-3-(*para*-aminophenyl)propanoyl]glycyl]-5-phenyl]-L-proline.

9. Process for the preparation of compounds according to claim 1, characterised in that the dipeptide of formula (II) : 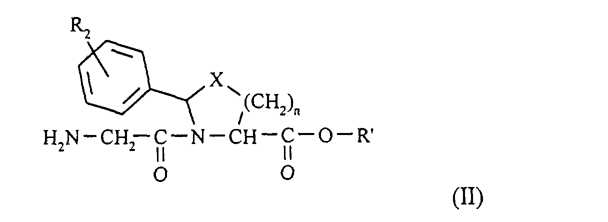 wherein R₂, R', X and n are as defined for the compound of formula (I), is subjected to acylation with an acid of formula (III) : 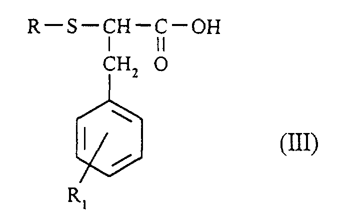 wherein R and R₁ are as defined for the compound of formula (I),
under the conditions customarily used in peptide synthesis,
the dipeptides of formula (II) being obtained by condensation of the Z-glycine acyl chloride of formula (IV) : 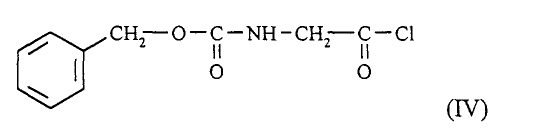 with an ester of formula (V) : 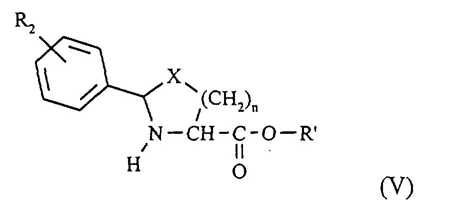 wherein R₂, R', X and n are as defined hereinbefore,
followed by catalytic hydrogenation.

10. Pharmaceutical compositions comprising as active ingredient at least one compound according to any one of claims 1 to 8 in combination with one or more pharmaceutically acceptable, inert, non-toxic carriers.

11. Pharmaceutical compositions according to claim 10 exerting a mixed inhibitory activity on neutral endopeptidase (NEP) and peptidyldipeptidase A (ACE) for use in the prevention and treatment of congestive heart failure and essential hypertension, hypertension associated with an increase in blood volume, atherosclerosis, cardiac ischaemia, cardiac and/or renal insufficiency, cor pulmonale and angina pectoris, and useful in their protective effect on the thickening of the vascular wall, as well as in their capacity to reduce the side-effects caused by treatment with cyclosporin.
